# EUROPÄISCHE PATENTSCHRIFT

(11) **EP 2 838 876 B1**
(45) Veröffentlichungstag und Bekanntmachung des Hinweises auf die Patenterteilung: **14.09.2016**
(21) Anmeldenummer: 13717023.9
(22) Anmeldetag: 12.04.2013
(51) Int. Cl.: C07C 209/36, C07C 209/84, C07C 211/46, C07C 211/47

(54) **VERFAHREN ZUM VERBESSERTEN ABFAHREN DER REAKTION BEI DER HERSTELLUNG VON AROMATISCHEN AMINEN AUS NITROAROMATEN**
METHOD FOR IMPROVED STOPPING OF THE REACTION WHEN PRODUCING AROMATIC AMINES FROM NITROAROMATICS
PROCÉDÉ DE DÉPART AMÉLIORÉ DE LA RÉACTION LORS DE LA FABRICATION D'AMINES AROMATIQUES À PARTIR DE NITRO-AROMATES

(30) Priorität: 16.04.2012 EP 12164324
(43) Veröffentlichungstag der Anmeldung: 25.02.2015
(73) Patentinhaber: Covestro Deutschland AG, 51373 Leverkusen (DE)
(72) Erfinder: MERKEL, Michael, 40223 Düsseldorf (DE); KNAUF, Thomas, 41542 Dormagen (DE); PETERS, Cliff Andre, 25724 Schmedeswurth (DE); SCHMIDT, Thorsten, 25704 Nindorf (DE); WILKE, Karl-Heinz, 47447 Moers (DE)
(74) Vertreter: Levpat
(86) Internationale Anmeldenummer: PCT/EP2013/057716
(87) Internationale Veröffentlichungsnummer: WO 2013/156409

(56) Entgegenhaltungen:
- EP-A1- 0 696 573
- EP-A2- 0 944 578

## Beschreibung

Die vorliegende Erfindung betrifft ein Verfahren zur Herstellung von aromatischen Aminen durch Hydrierung von aromatischen Nitroverbindungen, umfassend die Schritte des Zuführens von aromatischen Nitroverbindungen und Wasserstoff in einen Reaktor unter Kontaktierung eines Hydrierkatalysators und des Beendens des Zuführens von aromatischen Nitroverbindungen und Wasserstoff in den Reaktor.

Aromatische Amine sind wichtige Zwischenprodukte, die preiswert und in großen Mengen hergestellt werden müssen. Daher werden Produktionsanlagen für aromatische Amine in der Regel für sehr große Kapazitäten errichtet. Die hohe Produktivität dieser Anlagen ist durch sehr lange Reaktionszyklen und den störungsfreien Ablauf zwischen den An- und Abfahrvorgängen der Hydrierung zur Regeneration der eingesetzten Hydrierkatalysatoren gewährleistet.

Anilin ist wichtiges Zwischenprodukt z.B. zur Herstellung von Methylendiphenyldüsocyanat (MDI) und wird im großtechnischen Maßstab in der Regel durch katalytische Hydrierung von Nitrobenzol mit Wasserstoff hergestellt. Besonders bevorzugt sind Reaktionsführungen wie in GB 1 452 466 A1, EP 0 011 090 A1 oder EP 0 944 578 A2 (isotherme Fahrweise) und in EP 0 696 574 B1, EP 0 696 573 B1, EP 1 882 681 A1 (adiabate Fahrweise) beschrieben. Neben den genannten Verfahren mit stationären Katalysatorbetten sind auch solche mit fluidisierten Katalysatorbetten z. B. in DE 1114820 B, DE 1133394 B oder WO 2008/034770 A1 beschrieben.

Den beschriebenen adiabaten und isothermen Verfahren ist gemein, dass der Ausgangsstoff Nitrobenzol mit einem Überschuss an Wasserstoff umgesetzt wird.

Die Herstellung der aromatischen Amine erfolgt in Reaktionszyklen, weil die Katalysatoraktivität der Hydrierkatalysatoren stetig abnimmt.

Die Aktivität von gebrauchten Katalysatoren für die Hydrierung aromatischer Nitroverbindungen muss deshalb in periodischen Abständen wiederhergestellt werden. Dazu wird eine Regenerierung durchgeführt, indem kohlenstoffhaltige Ablagerungen vom Katalysator durch Abbrennen im Luftstrom entfernt werden. In anderen Ausführungsformen des Verfahrens wird der Abbrennstufe eine Waschstufe nachgeschaltet wie z. B. in US 3,684,740 beschrieben. Danach kann der nächste Reaktionszyklus gestartet werden, indem die Hydrieranlage wieder angefahren wird.

In einem adiabaten Verfahren zur Hydrierung von Nitroverbindungen zu den entsprechenden Aminen EP 0 944 578 A2 (S. 2, Z. 1-20) wird auf das Anfahrprocedere eingegangen. Dort wird beschrieben, dass es sich vorteilhaft auf die Raum-Zeit-Ausbeute auswirkt, die Belastung des Katalysators an eingesetzten aromatischen Nitroverbindungen kontinuierlich oder stufenweise innerhalb von 10 bis 1000 Stunden auf die maximale Belastung zu erhöhen.

Die Güte eines Verfahrens zur Hydrierung aromatischer Nitroverbindungen ist einerseits definiert durch den Gehalt des Produktes an unerwünschten Nebenprodukten der Reaktion. Andererseits ist die Güte eines Hydrierverfahrens dadurch definiert, dass der gesamte Prozess von Hydrierzyklus, Abfahren der Hydrierung, Regeneration des Hydrierkatalysators und das Anfahren des Hydrierprozesses ohne technischen Produktionsausfall betrieben werden kann.

In der Regel werden für heterogen katalysierte Gasphasenprozesse geträgerte Metallkatalysatoren eingesetzt, die ein bevorzugtes Temperaturfenster für ihre optimale Aktivität aufweisen. Wird die maximale Temperatur auch nur kurzzeitig überschritten, kann es zu einer Schädigung des Katalysators z.B. durch Sinterprozesse der aktiven Metallpartikel auf dem Trägermaterial kommen.

Den beschriebenen Verfahren des Standes der Technik gelingt es zwar mit hoher Selektivität und Ausbeute aromatische Amine herzustellen, jedoch werden mit Ausnahme von EP 0 944 578 A2, das das Anfahrprocedere beschreibt, nur Verfahren beschrieben, die schon in Gang sind und "Strich" laufen. Die möglichen Problematiken beim Abfahren eines Herstellungsprozesses von aromatischen Aminen werden übergangen.

Aufgabe der vorliegenden Erfindung war es daher ein Verfahren bereit zu stellen, welches ein reibungsloses Abfahren der Hydrierreaktion gewährleistet, und welches nicht zum Entstehen von unsicheren Anlagenzuständen oder zu Schädigungen des Hydrierkatalysators führt, so dass es beim Anfahren oder schon nach kurzen Hydrierzyklen zu einem Durchbruch des zu hydrierenden Nitroaromaten durch das Katalysatorbett kommt.

Erfindungsgemäß wird diese Aufgabe gelöst durch ein Verfahren zur Herstellung von aromatischen Aminen durch Hydrierung von aromatischen Nitroverbindungen, umfassend die Schritte:
A) Zuführen von aromatischen Nitroverbindungen und Wasserstoff in einen Reaktor unter Kontaktierung eines Hydrierkatalysators;
B) Beenden des Zuführens von aromatischen Nitroverbindungen und Wasserstoff in den Reaktor;
wobei Schritt B) derart durchgeführt wird, dass
B1) zuerst das Zuführen von aromatischen Nitroverbindungen in den Reaktor eingestellt wird, so dass im Reaktor und/oder in stromaufwärts mit dem Reaktor fluidisch verbundenen Anlagenteilen verbliebene aromatische Nitroverbindungen mit dem weiterhin zugeführten Wasserstoff reagieren und
B2) anschließend nach einer vorbestimmten Zeit und/oder nach Unterschreiten einer vorbestimmten Konzentration von aromatischen Nitroverbindungen im in den Reaktor eintretenden Gasstrom das Zuführen von Wasserstoff eingestellt wird.

Überraschend wurde gefunden, dass die Aufgabe für einen Hydrierprozess von aromatischen Nitroverbindungen gelöst werden kann, wenn (vereinfacht ausgedrückt und ohne hierauf beschränkt zu sein) beim Abfahren der Reaktion dafür gesorgt wird, dass Wasserstoff in genügender Menge in den Reaktionsräumen vorhanden ist bis sämtliche Nitroaromaten mit Wasserstoff abreagiert haben und somit aufgebraucht sind.

Einer der Vorteile dieser Vorgehensweise ist, dass kohlenstoffhaltige organische Verbindungen weitgehend aus dem vom Kreisgas durchströmten Reaktionsraum entfernt werden und deshalb die anschließende Regenerierung nicht mehr durch den Verbrauch von Sauerstoff stören können.

Vorzugsweise wird ein Reaktionszyklus abgefahren, indem zunächst die Zufuhr der Edukte (Nitroaromat und Wasserstoff) auf einen Bruchteil der Nennlast reduziert wird. Dann wird die Nitrobenzolzufuhr unter Beibehaltung aller anderen Fahrparameter komplett unterbrochen. Die Wasserstoffzufuhr bleibt solange in Betrieb, bis auch die letzten Reste des Nitroaromaten in den Reaktionsräumen verbraucht sind.

Stromaufwärts mit dem Reaktor fluidisch verbundene Anlagenteile sind insbesondere Zuleitungen, Sammelbehälter, Flansche, Verdampfer, Wärmetauscher, Überhitzer und dergleichen, in denen sich Reste von Nitroaromaten befinden können. Diese Reste würden beim Wiederanfahren in den Reaktor eingeschleppt werden.

Wenn die Reaktionsräume und vorzugsweise auch deren Peripherie vor dem Abfahren eines Reaktionszyklus zuerst von Nitroaromaten befreit werden, ergeben sich folgende Vorteile:
i) Es entsteht keine Übertemperatur auf dem Katalysator, was die Katalysatoraktivität beeinträchtigt bzw. die Produktivität der Anlage erniedrigt.
ii) Die Produktqualität leidet nicht wegen wiederholter Anfahrvorgänge, die typischerweise mit einer geringeren Anfangsselektivität einhergehen, bis sich stabile Prozessbedingungen eingestellt haben.
iii) Es wird vermieden, dass Nitrobenzolreste mit zu geringem Wasserstoffüberschuss hydriert werden, was zu verstärkter Nebenproduktbildung und Verkokung des Katalysators führt.
iv) Es entsteht kein Sicherheitsproblem, da in der Anlage kein Unterdruck entsteht. Gefahren des Unterdrucks sind mögliche Beschädigungen von nicht vakuumfesten Anlagenteilen und auch, dass Luft in die Anlage drückt und eine explosionsfähige Atmosphäre entstehen kann. Dieses muss in einer Hydrieranlage unbedingt vermieden werden.
v) Die Energiekosten für verlängerte/häufigere Regeneration und wiederholtes Anfahren werden eingespart.
vi) Verluste an Anilin durch Verbrennen während der Regeneration und damit verbundene längere Ausfallzeiten für die Regeneration werden weitgehend vermieden.

Insbesondere eignet es sich für das Abfahren der Hydrierung, wenn der Katalysator katalytisch aktive Komponenten auf einem Aluminiumoxid-Träger mit einem mittleren Durchmesser der Aluminiumoxid-Partikel zwischen 1,0 mm und 7,0 mm und einer BET-Oberfläche von weniger als 20 m²/g enthält, und bei dem die aktiven Komponenten mindestens umfassen:
(a) 1-100 g/l Träger mindestens eines Metalls der Gruppen 8 bis 12 des Periodensystems der Elemente, und
(b) 0 - 100 g/l Träger mindestens eines Übergangsmetalls der Gruppen 4 bis 6 und 12 des Periodensystems der Elemente, sowie
(c) 0 - 100 g/l Träger mindestens eines Metalls der Hauptgruppenelemente der Gruppen 14 und 15 des Periodensystems der Elemente.

Die Gruppen des Periodensystems der Elemente werden in dieser Druckschrift gemäß der IUPAC-Empfehlung von 1986 gezählt.

Der Aluminiumoxidträger hat bevorzugt annähernd Kugelform und bevorzugt einen Durchmesser im Bereich von 1,0 mm bis 7,0 mm.

Bevorzugte Reaktoren für einen isotherm betriebenen Reaktor sind thermostatisierte Rohr- oder Rohrbündelreaktoren. Geeignete Ausführungsformen solcher Reaktoren sind zum Beispiel in DE 2 201 528 A1, DE 2 207 166 A1, DE 198 06 810 A1, EP 1 439 901 A1, EP 1 569 745 A1, EP 1 590 076 A1, EP 1 587 612 A1, EP 1 586 370 A1, EP 1 627 678 A1 oder DE 202 006 014 116 U1 beschrieben.

Bevorzugte Reaktoren für einen adiabat betriebenen Reaktor sind die in DE 10 2006 035 203, Absätze [0030] bis [0033] beschriebenen.

Es ist möglich, dass der in dem Reaktor angeordnete Katalysator in einer radial durchströmten Filterkerze vorliegt. Dies kann zum Beispiel erreicht werden, indem der Katalysator in einem Korb gehalten wird, der aus zwei konzentrischen, zylindrischen Siebmänteln mit fluiddurchlässigen Wänden aufgebaut ist. Dabei weist ein Siebmantel einen größeren Radius auf als der andere, der auch als Zentralrohr bezeichnet wird, und der Raum zwischen den Siebmänteln ist der Reaktionsraum. Ein Boden dieses Hohlzylinders ist vorzugsweise komplett dicht verschlossen, während der andere nur bis an das Zentralrohr heran geschlossen ist, das an diesem Ende offen ist. Das Fluid kann nun in radialer Richtung von außen nach innen strömen und dann durch das Zentralrohr abgeführt werden. Alternativ kann das Fluid auch durch das Zentralrohr zugeführt werden und dann in radialer Richtung nach außen strömen, wo es dann abgeführt wird. Befindet sich dieser Reaktionsraum im gleichen Reaktor wie der isotherme Reaktionsraum, so ist er meist auf geeignete Weise mit dem Reaktorauslass verbunden.

Vorzugsweise wird die Hydrierung der aromatischen Nitroverbindungen gemäß Schritt A) im Rahmen des erfindungsgemäßen Verfahrens kontinuierlich betrieben.

Die vorliegende Erfindung wird anhand von Ausführungsformen weiter erläutert werden. Sie können beliebig miteinander kombiniert werden, sofern sich aus dem Zusammenhang nicht eindeutig das Gegenteil ergibt.

In einer Ausführungsform des erfindungsgemäßen Verfahrens wird in Schritt B1) das Wasserstoffgas durch den Reaktor zirkuliert. Es ist möglich, dass zusammen mit dem Wasserstoffgas weitere Gase mit zirkuliert werden. Die im rohen Reaktionsprodukt enthaltenen nichtkondensierbaren Gase werden dann in die Reaktion zurückgeführt. Diese Gase sind im Wesentlichen während der Hydrierung nicht umgesetzter Wasserstoff und gegebenenfalls zugesetzte oder durch Nebenreaktionen entstehende Inertgase. Es kann auch ein Teil des Kreisgases abgezweigt werden, beispielsweise, wenn dieses zum Konstanthalten der Konzentrationen weiterer gasförmiger Komponenten des Kreisgases erwünscht ist. Ein Beispiel hierfür ist auf dem Katalysator durch Desaminierungsreaktionen gebildetes Ammoniak.

Um die Zirkulation wenigstens des Wasserstoffgases zu erreichen, wird der den Reaktor verlassende Produktstrom soweit abgekühlt, dass die kondensierbaren Bestandteile möglichst vollständig kondensieren. Da der Kühlung aufgrund wirtschaftlicher Randbedingungen Grenzen gesetzt sind, kann nicht ausgeschlossen werden, dass geringe Mengen an kondensierbaren Bestandteilen in der Gasphase verbleiben. Diese werden dann zusammen mit dem Wasserstoff üblicherweise über einen Verdichter zurück in den Reaktor gefördert, so dass letztlich ein Wasserstoffkreislauf entsteht.

In einer weiteren Ausführungsform des erfindungsgemäßen Verfahrens wird vor Schritt B) die Menge an aromatischen Nitroverbindungen und Wasserstoff auf jeweils ≥ 1 Massen-% bis ≤ 40 Massen-% der zuvor in Schritt A) eingesetzten Menge reduziert. Vorzugsweise wird auf jeweils ≥ 2 Massen-% bis ≤ 20 Massen-% reduziert, mehr bevorzugt auf jeweils ≥ 5 Massen-% bis ≤ 15 Massen-%. Eine Reduktion der Menge an aromatischen Nitroverbindungen und Wasserstoff vor dem vollständigen Beenden ihrer Zufuhr vermeidet größere Temperatursprünge im Reaktor und damit Belastungen für das Material. Anderenfalls würde die Reaktionswärme plötzlich fehlen.

Weiterhin sollte die hier zugeführte Menge an Wasserstoff beim Abschalten der Nitroaromaten-Zufuhr ausreichen, um Nitroaromaten-Reste sicher abzureagieren, aber auch nicht zu groß sein, da der ganze eingebrachte und nicht umgesetzte Wasserstoff (einen konstanten Druck in der Anlage vorausgesetzt vorausgesetzt) als Purge entsorgt würde.

In einer weiteren Ausführungsform des erfindungsgemäßen Verfahrens wird nach Schritt B) der Reaktor mit einem Inertgas befüllt. Ein geeignetes Inertgas ist insbesondere Stickstoff. Durch das Inertisieren kann die Reaktionsanlage für die Regenerierung des Hydrierkatalysators vorbereitet werden.

Hinsichtlich der Betriebsweise des Reaktors bevorzugt sind Reaktionsführungen wie in EP 0 944 578 A2 (isotherme Fahrweise) und in EP 0 696 574 B1, EP 0 696 573 B1, EP 1 882 681 A1 (adiabate Fahrweise) beschrieben. Sie eignet sich besonders für das erfindungsgemäße Abfahren der Hydrierung. wenn die Katalysatoren, wie in US 3,684,740 beschrieben, regeneriert wurden.

In einer weiteren Ausführungsform des erfindungsgemäßen Verfahrens wird Schritt A) in isothermer Fahrweise betrieben. Diese Fahrweise hat den Vorteil einer geringeren thermischen (Wechsel-)Belastung des Reaktormaterials im Vergleich zur adiabaten Fahrweise.

In einer weiteren Ausführungsform des erfindungsgemäßen Verfahrens beträgt in Schritt A) das molare Verhältnis von Wasserstoff zu Nitrogruppen der aromatischen Nitroverbindungen ≥ 3:1 bis ≤ 6:1. Je geringer der Wasserstoffüberschuss im Hydrierprozess gewählt ist, desto größer sind die Vorteile durch das erfindungsgemäße Verfahren, da die negativen Effekte häufiger und/oder stärker auftreten.

In einer weiteren Ausführungsform des erfindungsgemäßen Verfahrens beträgt in Schritt B2) die vorbestimmte Zeit ≥ 10 Minuten bis ≤ 600 Minuten. Mehr bevorzugt beträgt die Zeit ≥ 30 Minuten bis ≤ 150 Minuten. Diese Zeitdauern stellen einen guten Kompromiss zwischen der Anforderung, dass sicher alle Nitroaromaten-Reste abreagiert sein sollen und andererseits die Anlage so schnell wie möglich regeneriert und wieder in den Produktionsbetrieb gebracht werden soll.

In einer weiteren Ausführungsform des erfindungsgemäßen Verfahrens beträgt in Schritt B2 die vorbestimmte Konzentration von aromatischen Nitroverbindungen im in den Reaktor eintretenden Gasstrom weniger als 1000 ppm, bevorzugt weniger als 100 ppm (Massen-ppm). Die Bestimmung der Konzentration kann zum Beispiel mittels zuvor kalibrierter online-Gaschromatographie erfolgen.

In einer weiteren Ausführungsform des erfindungsgemäßen Verfahrens werden in Schritt A) aromatische Nitroverbindungen gemäß der allgemeinen Formel (I) eingesetzt: 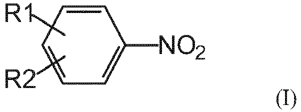 in der R1 und R2 unabhängig voneinander Wasserstoff, Methyl oder Ethyl bedeuten, wobei R2 weiterhin auch NO₂ bedeuten kann. Bevorzugte aromatische Amine sind Nitrobenzol und Dinitrotoluol, so dass durch deren Hydrierung Anilin beziehungsweise Toluylendiamin erhalten wird.

In einer weiteren Ausführungsform des erfindungsgemäßen Verfahrens ist im Reaktor der Katalysator in einem festen Katalysatorbett angeordnet. Neben den genannten Verfahren mit stationären Katalysatorbetten sind auch solche mit fluidisierten Katalysatorbetten z. B. in DE 1114820 B, DE 1133394 B oder WO 2008/034770 A1 beschrieben. Alternativ kann der Katalysator auch in einem fluidisierten Bett vorliegen.

Anhand der nachfolgenden Beispiele soll die vorliegende Erfindung näher beschrieben werden, ohne jedoch darauf beschränkt zu sein.

Beispiel 1 (Vergleichsbeispiel isotherm): Abfahren der Hydrierung von Nitrobenzol in einer Anlage am Ende eines Reaktionszyklus, in dem die Wasserstoffzufuhr und Nitrobenzolzufuhr gleichzeitig abgestellt werden. Folge: Auftreten eines unerwünschten Vakuums im Reaktionsraum.

In einem mittels Marlotherm® - Kreislauf thermostatisierten Rohrbündelreaktor zur Hydrierung von Nitrobenzol wurden Nitrobenzol und Wasserstoff im molaren Verhältnis von 1:6 zur Reaktion gebracht. Als Katalysator diente ein geträgerter Edelmetallkatalysator, wie er in DE-OS2849002, Beispiel 1 beschrieben ist und der als Schüttung in die Rohre des Reaktors eingebracht wurde. Der überschüssige Wasserstoff wurde in der Kondensation der Reaktionsprodukte abgetrennt, zurückgewonnen und wieder in die Reaktion eingesetzt (Kreisgas-Wasserstoff).

Die kondensierten Reaktionsprodukte wurden aus dem Reaktionssystem entfernt und einer Aufarbeitung zugeführt. Verbrauchter Wasserstoff wurde durch frischen Wasserstoff ergänzt (Frisch-Wasserstoff). Zur Kreisführung des zurückgewonnenen Wasserstoffs diente ein Verdichter. Er sog den Wasserstoff aus den Kondensatoren an und förderte ihn in die Nitrobenzol-Verdampfung zurück.

Um zu verhindern, dass sich inerte Komponenten im Kreisgas anreichern, wurde ein Seitenstrom als Purge abgezweigt. Dies erfolgte über ein Regelventil (alternativ wäre eine Tauchung möglich), das auch als Druckhaltung für das Reaktionssystem diente.

Zum Beenden der Reaktion wurde die Zufuhr von Nitrobenzol und Frischwasserstoff gleichzeitig unterbrochen. Der Anlagendruck wurde dabei auf der Saugseite des Verdichters gemessen und verfolgt. Die Kreisgasverdichter blieben während des gesamten Abfahrvorgangs in Betrieb. Die Anlage konnte nun durch Inertisierung mit Stickstoff für eine anschließende Regenerierung und Wiederanfahrt vorbereitet werden. Die gemessenen Drücke sind in der nachfolgenden Tabelle wiedergegeben. Man erkennt, dass sich im Laufe der Zeit nach Unterbrechen der Eduktversorgung ein Unterdruck im Reaktor aufbaut.

| **Zeitpunkt:** | Während der Reaktion | Unterbrechen der Eduktversorgung | 5 Min. nach Unterbrechen der Eduktversorgung | 30 Min. nach Unterbrechen der Eduktversorgung |
|---|---|---|---|---|
| **Druck auf der Saugseite des Verdichters:** | 1,1 bar (a) | 1,1 bar (a) | 1,05 bar (a) | 0,95 bar (a) |

Beispiel 2 (erfindungsgemäßes Beispiel isotherm): In einem Prozess gemäß Beispiel 1 wurde zum Abfahren der Hydrierung von Nitrobenzol am Ende eines Reaktionszyklus zunächst die Belastung mit Edukten (Nitrobenzol und Wasserstoff) auf 10% der Nennlast reduziert.

Ausgehend von diesem Zustand wurde die Nitrobenzolzufuhr unter Beibehaltung aller anderen Fahrparameter komplett unterbrochen. Die Wasserstoffzufuhr blieb nach Unterbrechung der Nitrobenzolzufuhr noch eine Stunde lang in Betrieb und wurde dann ebenfalls vollständig unterbrochen. Die Kreisgasverdichter blieben während des gesamten Anfahrvorgangs in Betrieb. 30 Minuten nach Unterbrechen der Wasserstoffzufuhr wurde noch einmal der Druck in der Anlage kontrolliert, bevor die Anlage durch Inertisierung mit Stickstoff für eine anschließende Regenerierung und Wiederanfahrt vorbereitet wurde.

Die gemessenen Drücke sind in der nachfolgenden Tabelle wiedergegeben. Der Druck im Reaktor blieb nach Unterbrechen der Eduktversorgung stabil.

| **Zeitpunkt:** | Während der Reaktion | Unterbrechen der Nitrobenzol-Versorgung | 30 Min. nach Unterbrechen der Nitrobenzol-Versorgung | 90 Min. nach Unterbrechen der Nitrobenzol-Versorgung (30 Min. nach Unterbrechen der H₂-Versorgung) |
|---|---|---|---|---|
| **Druck auf der Saugseite des Verdichters:** | 1,1 bar (a) | 1,1 bar (a) | 1,1 bar (a) | 1,09 bar (a) |

## Patentansprüche

1. Verfahren zur Herstellung von aromatischen Aminen durch Hydrierung von aromatischen Nitroverbindungen, umfassend die Schritte:
A) Zuführen von aromatischen Nitroverbindungen und Wasserstoff in einen Reaktor unter Kontaktierung eines Hydrierkatalysators;
B) Beenden des Zuführens von aromatischen Nitroverbindungen und Wasserstoff in den Reaktor;
**dadurch gekennzeichnet, dass** Schritt B) derart durchgeführt wird, dass
B1) zuerst das Zuführen von aromatischen Nitroverbindungen in den Reaktor eingestellt wird, so dass im Reaktor und/oder in stromaufwärts mit dem Reaktor fluidisch verbundenen Anlagenteilen verbliebene aromatische Nitroverbindungen mit dem weiterhin zugeführten Wasserstoff reagieren und
B2) anschließend nach einer vorbestimmten Zeit und/oder nach Unterschreiten einer vorbestimmten Konzentration von aromatischen Nitroverbindungen im in den Reaktor eintretenden Gasstrom das Zuführen von Wasserstoff eingestellt wird.

2. Verfahren gemäß Anspruch 1, wobei in Schritt B1) das Wasserstoffgas durch den Reaktor zirkuliert wird.

3. Verfahren gemäß Anspruch 1, wobei vor Schritt B) die Menge an aromatischen Nitroverbindungen und Wasserstoff auf jeweils ≥ 1 Massen-% bis ≤ 40 Massen-% der zuvor in Schritt A) eingesetzten Menge reduziert wird.

4. Verfahren gemäß Anspruch 1, wobei nach Schritt B) der Reaktor mit einem Inertgas befüllt wird.

5. Verfahren gemäß Anspruch 1, wobei Schritt A) in isothermer Fahrweise betrieben wird.

6. Verfahren gemäß Anspruch 1, wobei in Schritt A) das molare Verhältnis von Wasserstoff zu Nitrogruppen der aromatischen Nitroverbindungen ≥ 3:1 bis ≤ 6:1 beträgt.

7. Verfahren gemäß Anspruch 1, wobei in Schritt B2) die vorbestimmte Zeit ≥ 1 Stunde beträgt.

8. Verfahren gemäß Anspruch 1, wobei in Schritt B2 die vorbestimmte Konzentration von aromatischen Nitroverbindungen im in den Reaktor eintretenden Gasstrom weniger als 1000 ppm beträgt.

9. Verfahren gemäß Anspruch 1, wobei in Schritt A) aromatische Nitroverbindungen gemäß der allgemeinen Formel (I) eingesetzt werden: 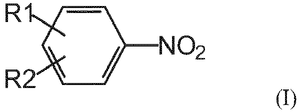 in der R1 und R2 unabhängig voneinander Wasserstoff, Methyl oder Ethyl bedeuten, wobei R2 weiterhin auch NO₂ bedeuten kann.

10. Verfahren gemäß Anspruch 1, wobei im Reaktor der Katalysator in einem festen Katalysatorbett angeordnet ist.

## Claims

1. Process for preparing aromatic amines by hydrogenation of aromatic nitro compounds, which comprises the steps:
A) introduction of aromatic nitro compounds and hydrogen into a reactor with contacting of a hydrogenation catalyst;
B) stopping of the introduction of aromatic nitro compounds and hydrogen into the reactor;
**characterized in that** step B) is carried out by
B1) firstly stopping the introduction of aromatic nitro compounds into the reactor so that aromatic nitro compounds remaining in the reactor and/or in plant parts fluidically connected upstream to the reactor react with the hydrogen which continues to be fed in and
B2) subsequently stopping the introduction of hydrogen after a predetermined time and/or after the concentration of aromatic nitro compounds in the gas stream entering the reactor goes below a predetermined value.

2. Process according to Claim 1, wherein the hydrogen gas is circulated through the reactor in step B1).

3. Process according to Claim 1, wherein the amount of aromatic nitro compounds and hydrogen is reduced in each case to from ≥ 1% by mass to ≤ 40% by mass of the amount introduced previously in step A) before step B).

4. Process according to Claim 1, wherein the reactor is filled with an inert gas after step B).

5. Process according to Claim 1, wherein step A) is carried out isothermally.

6. Process according to Claim 1, wherein the molar ratio of hydrogen to nitro groups of the aromatic nitro compounds in step A) is from ≥ 3:1 to ≤ 6:1.

7. Process according to Claim 1, wherein the predetermined time in step B2) is ≥ 1 hour.

8. Process according to Claim 1, wherein the predetermined concentration of aromatic nitro compounds in the gas stream entering the reactor in step B2 is less than 1000 ppm.

9. Process according to Claim 1, wherein aromatic nitro compounds having the general formula (I): 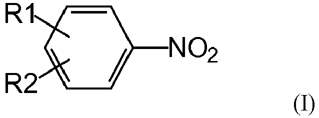 where R1 and R2 are each, independently of one another, hydrogen, methyl or ethyl and R2 can also be NO₂, are used in step A).

10. Process according to Claim 1, wherein the catalyst is arranged in a fixed catalyst bed in the reactor.

## Revendications

1. Procédé pour la préparation d'amines aromatiques par hydrogénation de composés nitro aromatiques, comprenant les étapes consistant à :
A) introduire des composés nitro aromatiques et de l'hydrogène dans un réacteur avec mise en contact avec un catalyseur d'hydrogénation ;
B) terminer l'introduction des composés nitro aromatiques et de l'hydrogène dans le réacteur ;
**caractérisé en ce que** l'étape B) est réalisée de manière telle que
B1) l'introduction de composés nitro aromatiques dans le réacteur est d'abord réglée de manière telle que des composés nitro aromatiques restant dans le réacteur et/ou dans les parties d'installation reliées en aval, de manière fluidique, avec le réacteur réagissent avec l'hydrogène en outre introduit et
B2) ensuite, après un temps prédéterminé et/ou après le passage sous une concentration prédéterminée en composés nitro aromatiques, l'introduction d'hydrogène est réglée dans le flux gazeux entrant dans le réacteur.

2. Procédé selon la revendication 1, l'hydrogène gazeux étant mis en circulation à travers le réacteur dans l'étape B1).

3. Procédé selon la revendication 1, la quantité de composés nitro aromatiques et d'hydrogène étant réduite, avant l'étape B), à chaque fois à ≥ 1% en masse jusqu'à ≤ 40% en masse de la quantité utilisée au préalable dans l'étape A).

4. Procédé selon la revendication 1, le réacteur étant rempli par un gaz inerte après l'étape B).

5. Procédé selon la revendication 1, l'étape A) étant réalisée dans un mode opératoire isothermique.

6. Procédé selon la revendication 1, le rapport molaire d'hydrogène à groupes nitro des composés nitro aromatiques dans l'étape A) étant ≥ 3:1 à ≤ 6:1.

7. Procédé selon la revendication 1, le temps prédéterminé dans l'étape B2) étant ≥ 1 heure.

8. Procédé selon la revendication 1, la concentration prédéterminée en composés nitro aromatiques dans le flux gazeux entrant dans le réacteur dans l'étape B2) étant inférieure à 1000 ppm.

9. Procédé selon la revendication 1, des composés nitro aromatiques selon la formule générale (I) étant utilisés dans l'étape A) : 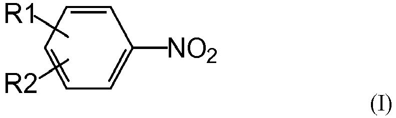 dans laquelle R1 et R2 signifient, indépendamment l'un de l'autre, hydrogène, méthyle ou éthyle ou R2 pouvant également signifier en outre NO₂.

10. Procédé selon la revendication 1, le catalyseur étant disposé dans le réacteur dans un lit catalytique fixe.
